# EUROPEAN PATENT APPLICATION

(11) **EP 4 585 133 A1**
(43) Date of publication of application: **16.07.2025**
(21) Application number: 24151614.5
(22) Date of filing: 12.01.2024
(51) Int. Cl.: A61B 1/00, A61B 1/04, A61B 5/00

(54) **CAPSULE ENDOSCOPY SYSTEM**

(71) Applicant: Ovesco Endoscopy AG, 72076 Tübingen (DE)
(72) Inventor: SCHOSTEK, Sebastian, 72116 Mössingen (DE); KELLER, Jan, 72074 Tübingen (DE); SCHURR, Marc Oliver, 72072 Tübingen (DE)
(74) Representative: Winter, Brandl - Partnerschaft mbB

(57) **Abstract**

The disclosure is directed to a capsule endoscopy system comprising a capsule endoscope (1), the capsule endoscope (1) comprising a first sensor module/unit (2), in particular an imaging system and an illumination source, for acquiring sensor data, a location detector (3) being configured to track a location of the capsule endoscope, and an association unit (4) being configured to associate the acquired sensor data with the location of the capsule endoscope.

## Description

The present invention relates to a capsule endoscopy system comprising a capsule endoscope/medical capsule/sensor capsule.

A capsule endoscope is a medical device that is used to examine a gastrointestinal tract of a human body of a patient. The capsule endoscope is a wireless device that is swallowed by the patient and transported substantially by the natural peristaltic motion of the gastrointestinal tract through the patient's body. Hence, the capsule endoscope follows the natural path, passes through the entire gastrointestinal tract and is excreted naturally at the end of the gastrointestinal tract.

The capsule endoscope comprises a sensor module that is configured to acquire data of the gastrointestinal tract while passing through the gastrointestinal tract. There are capsule endoscopes, so-called imaging sensor capsules, comprising an imaging system, in particular a camera device and an illumination source, as the sensor module for acquiring images of an inner surface of the gastrointestinal tract. There are capsule endoscopes, so-called non-imaging sensor capsules, comprising another sensor except for an imaging system, e.g. an acidity sensor, a pressure sensor, a temperature sensor, a blood detection sensor, or a bile sensor, as the sensor module for acquiring respective sensor data of the gastrointestinal tract. Further, there are capsule endoscopes comprising more than one sensor module, for example, comprising an imaging system as a first sensor module as well as another sensor (except for an imaging system) as a second sensor module. In general, the disclosure refers to all type of capsule endoscopes.

The capsule endoscope is preferably shaped like a pill, meaning that the geometry essentially consists of a cylinder with two hemispheres at either end. Variations of this geometry, such as a cylinder with a non-circular base or hemispheres with a non-circular profile or a cylinder with two spherical sectors (less than hemispheres) at either end, are also possible. The capsule endoscope has a longitudinal axis essentially parallel (in particular corresponding) to an axis of the cylinder shape. The hemispherical shapes of the geometry are placed at the front and the back of the capsule endoscope.

The capsule endoscopy system, in particular the capsule endoscope, performs a wireless measurement inside the body of the patient for diagnostic purposes. Such a measurement can be positive or negative. Positive means that a pathology has been detected/found and negative means that no pathology has been detected/found.

However, in either case, there may be several technical limitations prohibiting a proper/adequate correlation between the finding/diagnosis and further clinical actions to be taken. In particular, due to insufficient (sensoric) equipment and/or due to unfavourable conduction of the capsule endoscopy/unfavourable operational settings of the capsule endoscope, a pathology may be not at all or not sufficiently/completely detected and recorded. This may lead to requiring additional follow-up examinations for the acquisition of data that could actually already have been acquired with the capsule endoscope, or repetition of the capsule endoscopy, or even unnecessary or pointless follow-up examinations, e.g. performed in the wrong place or at a time when the pathology has changed, moved or disappeared. All such circumstances may increase burden of the patient, workload for healthcare professionals and cost for healthcare providers / payers and reduced efficiency of healthcare delivery due to e.g. delayed diagnosis and treatment.

Thus, it is the object of the disclosure to provide a capsule endoscopy system that allows relevant data to be efficiently collected and recorded, and the collected data to be reliably and promptly analyzed to enable appropriate clinical actions to be taken with minimal patient discomfort.

The object of the disclosure is solved by a capsule endoscopy system with the features according to claim 1. Advantageous embodiments are subject matter of the dependent claims.

Accordingly, the disclosure is directed to a capsule endoscopy system comprising a capsule endoscope. The capsule endoscope is preferably configured to be swallowable by a patient for passing through a gastrointestinal tract of a human body of the patient.

The capsule endoscope comprises a first sensor module/unit, in particular an imaging system, preferably a camera device and an illumination source, for acquiring sensor data, in particular images of an inner surface, of the gastrointestinal tract of the patient.

The capsule endoscope comprises a location detector being configured to track a location, preferably an anatomical location, of the capsule endoscope. That is, the anatomical location (in contrast to a Cartesian location) refers to the location along the anatomy of the gastrointestinal tract, such as the organ segment (mouth, esophagus, stomach, duodenum, jejunum, ileum, colon, rectum) or the position within such organ segments, i.e. the travel distance within the organ. Further, since a large part of the bowel is rather loosely suspended, organ segments such as a small bowel segment can change position within the abdominal cavity. Therefore, the Cartesian coordinates of a finding cannot be directly associated with the anatomical location as the organ segment has likely shifted significantly within the abdominal cavity over time. Therefore, it is preferred to track the anatomical location rather than the Cartesian coordinates (x-, y-, z-coordinates in a coordinate system fixed to the patient's body) with the location detector.

The capsule endoscope comprises an association unit. The association unit is configured to associate the sensor data acquired with the first sensor module/unit, in particular the acquired images, with the (anatomical) location of the capsule endoscope. The association unit may be a data processing unit that establishes the relation between the (anatomical) location determined by the location detector with sensor data acquired with the first sensor module/unit by e.g. attributing identifiers, time stamps or grouping data accordingly.

In other words, the capsule endoscope according to the disclosure does not only measure sensor data with its first sensor module/unit which have to be analyzed at a later stage, but already establishes the relation between the sensor data acquired with the first sensor module/unit and the (anatomical) location determined with the location detector.

Thus, the capsule endoscope allows for evaluating the sensor data acquired with the first sensor module/unit in the light of where it was found (as a particular appearance may be normal or conspicuous/unusual depending on the location) in order to allow the clinician / physician to make an clinically appropriate assessment and to take appropriate reaction to it. In particular, in case of a finding or unusual/positive result, it may remain unclear at what location in the gastrointestinal tract the finding or result is located. If the information of the location is missing, it may lead to unnecessary clinical investigations, for example, such as a transoral device-assisted enteroscopy (DAE) while the finding is located too distal in the gastrointestinal tract, e.g. in the small bowel, to be reached transorally. If the location would have been known, the patient could have been spared a transoral procedure and the finding could have been reached with DAE in a transanal approach. Hence, tracking the location and associating the (anatomical) location determined with the location detector with the sensor data acquired with the first sensor module/unit can help to interpret the data correctly and take adequate clinical actions.

In general, time delay should be avoided as it may lead to clinically false (positive) results, since a finding, for example, such as a bleeding lesion might have been disappeared when a therapeutic endoscopy is performed several hours or days after the detection of the lesion by the capsule endoscope. Further, in case of a time delay, the patient often has already resumed normal diet such that, for further examination, another bowel cleansing procedure is required and thus, a further delay of often 36 hours as well as additional patient discomfort is generated.

According to a preferred embodiment, the location detector may be a movement detector/tracer, a near-field imaging system, an accelerometer, a magnetometer, a pH sensor, a bile sensor/detector or a temperature sensor. Alternatively or additionally, the location detector may be a data processing unit being configured to determine the (anatomical) location from the sensor data acquired with the first sensor module/unit, in particular form the images of the inner surface of the gastrointestinal tract. The location detector may also be a combination of said sensor types and/or said data processing unit. In other words, the location detector may be a sensor device that itself detects/measures a specific sensor parameter indicative for a specific organ segment, or a processing unit that accesses/uses/extracts existing (sensor) data, in particular images, and determines the (anatomical) location from these existing (sensor) data through data processing.

A near-field imaging system comprises at least a camera and an illumination that is configured to obtain close-up images of a gastrointestinal wall tissue or an organ content, for example images that cover about 1 mm² of a gastrointestinal organ wall (1 mm x 1 mm) to show superficial structure such as intestinal villi. In contrast to imaging systems designed to obtain full coverage of the entire gastrointestinal wall surface, near-field imaging systems can only acquire a small fraction of imaging data e.g. in a point-to-point manner. Such near-field imaging systems are used to obtain information about the structural or superficial patterns of gastrointestinal wall tissue in order to determine the organ section by means of the different tissue appearance (e.g. presence of intestinal villi) or to detect movement by comparing differences between two subsequent frames using an additional processing unit of the near-field imaging system. When used in conjunction with a processing unit for motion detection, it is also referred to as movement detector / tracer.

According to a further aspect, which may be present in combination with or independently of the above-described aspect of the capsule endoscope comprising a location detector, the capsule endoscope may comprise an abnormality detector. The abnormality detector is configured to detect an abnormality in the gastrointestinal tract. The abnormality may be indicative of a pathology. For example, the presence of blood in the lumen of the gastrointestinal tract may be indicative for a bleeding lesion in the gastrointestinal tract. In this example, the presence of blood represents an abnormality, while the bleeding lesion represents a pathology. Both are measured / detected with different (kinds of) sensor devices. Another example is that a too high or too low or locally increased or locally decreased temperature may be indicative of another pathology such as a local/regional inflammatory disease. In other words, the capsule endoscope comprises an additional sensor/detector. As in case of a negative result, it may remain unclear if a pathology does not exist or if it was just not detected by the first sensor module/unit. In particular, the completeness of the inspection of (the target segments of) the gastrointestinal tract may not be recorded by the capsule endoscopy system itself. Thus, by equipping the capsule endoscope with the abnormality detector and simultaneously recording and associating/assigning the location to the recorded/acquired sensor data, the probability of recognizing/detecting the pathology during the capsule endoscopy and being able to react to it immediately and appropriately is greatly increased.

The abnormality detector may be a blood detector/sensor, a bile detector/sensor, a temperature detector/sensor, a pH detector/sensor, a gas sensor, a movement detector/tracer, an accelerometer, a magnetometer, a near-field imaging system, or another detector/sensor. It may also be a sensor configured to detect the presence of specific markers in the gastrointestinal lumen, such as a fluorescence sensor configured to detect the presence of a fluorescent agent such as fluorescein or the like. The abnormality detector may also be a combination of said sensor types and/or said data processing unit. Alternatively or additionally, the abnormality detector may be a data processing unit being configured to detect an abnormality based on the acquired sensor data of the first sensor unit, in particular of the acquired images (of the inner surface) of the gastrointestinal tract. In other words, the abnormality detector may be a sensor device that itself detects/measures a specific sensor parameter (and compares it with limit/threshold values, if applicable), or a processing unit that accesses/uses/extracts existing (sensor) data and determines an abnormality from these existing (sensor) data through data processing.

According to a preferred embodiment, the association unit may be configured to associate the sensor data acquired with the abnormality detector with the sensor data acquired with the first sensor module/unit, in particular the acquired images. Preferably, the association unit may be configured to additionally associate the sensor data acquired with the abnormality detector with the anatomical location of the capsule endoscope determined with the location detector. That is, the association unit establishes a relation between the sensor data acquired with the first sensor module/unit, in particular images, and the sensor data concerning the abnormality, and preferably additionally with the anatomical location of the abnormality. This makes the data more meaningful and makes it easier to check at a later stage whether the diagnosis of the pathology was correct.

According to a preferred embodiment, the association unit may be configured to only associate the relation between the (anatomical) location of the capsule endoscope determined by the location detector and the sensor data acquired with the first sensor module/unit in case of a detection of an abnormality. Thus, this allows the amount of data to be reduced to that which is necessary to assess the abnormality.

According to a preferred embodiment, the capsule endoscope at least comprises an imaging system as the first sensor module/unit, a temperature sensor as the location detector and a blood detector/sensor as the abnormality detector. When the patient regularly swallows liquid the temperature of which is different to body temperature, this configuration allows for determining whether the capsule endoscope is located in the stomach or not, hence if the capsule is located in the stomach or already passed the pylorus when an abnormality is detected by the abnormality detector, or a lesion is identified by the sensor data acquired with the first sensor module/unit. If the capsule endoscope is in the stomach, ingestion of liquid/food of different temperatures leads to a notable increase or decrease in temperature sensor signal; on the other hand, if the capsule endoscope already passed the pylorus, the ingestion of such liquid/ food will not lead to a notable change in temperature sensor output.

Alternatively or additionally, the capsule endoscope may also be equipped with a bile detector sensor and/or pH detector sensor as the location detector in order to determine the passage of the pylorus by sensing the exposure to bile juice either through a bile detector (e.g. optical sensor sensitive to colorants in the bile) or the increase of pH value relative to the intragastic pH value. A temperature sensor would further indicate excretion of the capsule endoscope through a drop in temperature after leaving the patient's body.

Alternatively or additionally, the capsule endoscope may also be equipped with a gas sensor or fluorescence sensor as the abnormality detector in order to determine abnormal concentrations of markers and/or other substances in the lumen of the gastrointestinal tract that can be considered abnormal, e.g. certain gas levels of methane or hydrogen that could be indicative of small intestinal bacterial overgrowth or other pathologies. Example of specific markers are fluorescent markers such as intravenously administered Fluorescein the intraluminal presence of which could be indicative of a compromised gut barrier.

According to another preferred embodiment, the capsule endoscope at least comprises an imaging system as the first sensor module/unit, an accelerometer, preferably a 3-axis accelerometer, and a magnetometer, preferably a 3-axis magnetometer, as the location detector and a data processing unit as the abnormality detector. The imaging system may be configured as near-field imaging system in order to acquire close-up images of the gastrointestinal wall, e.g. for primary diagnostic purposes such as evaluation of the intestinal villi, e.g. in case of suspected villous atrophy. An accelerometer and magnetometer serve as location detector by observing capsule movement with respect to the gravity vector and the magnetic field of the earth or any other source of magnetic field, and identifying the location of the capsule endoscope by means of the movement patterns observed. The movement patterns differ from organ segment to organ segment, e.g. the capsule endoscope is able to freely rotate in the large lumen of the stomach while it is essentially guided in the small intestine, in particular the jejunum and ileum, due to the smaller caliber of this organ segment, thus not being able to rotate freely about its longitudinal axis with respect to the organ lumen. The accelerometer and magnetometer can be used with the gravity vector and earth magnet field as reference, or in conjunction with an accelerometer and magnetometer that is mounted / fixed to the patient's trunk in order to provide a reference to the patient's body position and posture. The data processing unit that serves as the abnormality detector may obtain information about peristaltic movements and/or travel patterns of the capsule along the gastrointestinal tract, in particular the small bowel by analyzing the movement distance between two subsequent frames obtained by the first sensor module/unit. The travel pattern may therefore indicate e.g. gastrointestinal abnormalities in motility such as very slow high fast or uncoordinated peristaltic movements.

Alternatively or additionally, the capsule endoscope may also be equipped with a data processing unit as the location detector by analyzing subsequent frames of the first sensor module/unit in order to determine the travel distance in particular in the small bowel.

Alternatively or additionally, the capsule endoscope may also be equipped with an accelerometer and a magnetometer as the abnormality detector by obtaining movement patterns with respect to the gravity vector and an extracorporeal magnetic field such as the earth magnetic field in order to identify abnormalities in gastrointestinal motility.

According to another preferred embodiment, the capsule endoscope at least comprises an imaging system as the first sensor module/unit, a data processing unit as the location detector and a blood sensor as the abnormality detector. The imaging system is configured to acquire images from the lumen of the gastrointestinal tract, preferably as front and/or backwards oriented imaging system with reference to the longitudinal axis of the capsule endoscope, in order to facilitate endoscopic visual examination. The acquired images are made available to the clinical user / physician. The capsule endoscope further comprises the data processing unit as the location detector by processing images acquired by the first sensor module/unit for determining the organ segment by means of the content of the image. This can be obtained by e.g. providing reference images and/or equipping the processing unit with e.g. a neural network that was trained for the detection of the various gastrointestinal tract segments by means of the image content. The data processing unit may determine the anatomical location of the capsule endoscope as e.g. "esophagus", "stomach", "duodenum", "jejunum", "ileum", "colon". The capsule endoscope further comprises the blood sensor as the abnormality detector for determining the presence of blood in the lumen of the gastrointestinal tract as abnormality and to indicate the presence of a pathology in the gastrointestinal tract.

Alternatively or additionally, the capsule endoscope may also be equipped with a bile sensor, a pH sensor, a temperature sensor or a combination of an accelerometer, preferably a 3-axis accelerometer, and a magnetometer, preferably a 3-axis magnetometer, as the location detector.

Alternatively or additionally, the capsule endoscope may also be equipped with a data processing unit as the abnormality detector for analyzing the content of the images acquired by the first sensor module/unit for the detection of abnormalities. This can be obtained by e.g. providing reference images and/or equipping the processing unit with e.g. a neural network that was trained for the detection of the various gastrointestinal tract segments by means of the image content.

Alternatively or additionally, the capsule endoscope may also be equipped with a bile sensor, pH sensor, fluorescence sensor, a gas sensor, a combination of an accelerometer, preferably a 3-axis accelerometer, and a magnetometer, preferably a 3-axis magnetometer, or any other type of sensor as the abnormality detector.

There may be more preferred embodiments of capsule endoscopes and their combination / configuration of first sensor module/unit, location detector and abnormality detector which are not described in this disclosure.

According to a preferred embodiment, the capsule endoscopy system may comprise a control unit being configured to control an operation mode of the first sensor module/unit, in particular the imaging system. The operation mode may be a measurement interval of the first sensor module/unit, in particular a frame rate of the imaging system, or a setting of the first sensor module/unit, in particular a resolution or an illumination brightness of the imaging system. Thus, the acquired sensor data, preferably the quality of the acquired sensor data, can be actively controlled and influenced, preferably improved.

The control unit may also be configured to control an operation mode of modules of the capsule endoscope other than the first sensor module/unit. For example, the control unit may be configured to control an operation mode of the location detector, the association unit and/or the abnormality detector.

Further, the capsule endoscope may comprise a data transmission unit for (wirelessly) transmitting the acquired data to an external processing device, in particular to an extracorporeal onbody device. Thus, the acquired data can be sent while the capsule endoscope is in the patient's body, preferably in real time, via radio/wirelessly to the external processing device, in particular the extracorporeal onbody device and evaluated.

Further, the capsule endoscope may comprise an internal data storage unit for storing the acquired data. Thus, the acquired data can be stored in the internal data storage unit, that is, in capsule endoscope itself, and, after the capsule endoscope has been excreted, the acquired data can be read out and evaluated.

Further, the capsule endoscope may comprise an energy source, in particular a battery, for providing the sensor modules, detector modules and/or the data storage unit and/or data transmission unit with energy. The energy source may comprises at least one regulator device for electrical energy regulation such as for stabilization and/or device activation.

The control unit may also be configured to control an operation mode of the energy source, the internal data storage unit, (the transmission function of) the data transmission unit, and/or any other module/unit incorporated into the capsule endoscope.

According to a preferred embodiment, the control unit is configured to change the operation mode of the first sensor module/unit in case an abnormality is detected by the abnormality detector. In particular, the control unit may be configured to adapt the resolution or illumination brightness and/or to increase the frame rate or alter the mode of operation of the capsule endoscope in any other way in order to achieve more accurate results. In contrast to known capsule endoscopy systems where a compromise between maintaining constant performance over the entire capsule endoscopy (taking several hours) and limited power available has to be chosen such that a high performance level cannot be maintained through the entire procedure, in the capsule endoscopy system according to the disclosure an abnormality detection leads to an adaptation of the capsule endoscope's mode of operation. Hence, the capsule's mode of operation can be adapted to a higher performance level which would not be able to maintain throughout the entire procedure, but for a certain fraction of the procedure time when diagnostic data of higher quality is desirable.

In other words, the capsule endoscopy system according to the disclosure solves the problem that a large part of the energy is consumed by acquiring and processing image/sensor data without a clinically significant finding, leaving only a small fraction of energy to be consumed by acquisition and processing of image/sensor data with clinically significant finding. The quality of image/sensor data with a clinically significant finding is thus limited due to the limited energy left. Since the size of the capsule is very limited, the size of the battery is also limited. Thus, even though data storage components are becoming smaller and can store larger amounts of data, it is not desirable to expand the amount of data generated excessively, as not only storage consumes energy, but especially wireless transmission through tissue is very energy-consuming. Hence, it is disadvantageous to produce many data on inconspicuous/normal sites, e.g. redundant or meaningless/irrelevant data or high-resolution data, and too little data on potentially pathological sites. Thus, the selectively executed change of the operation mode improves the quality and the information value of the acquired data, especially those data with a clinically significant finding.

For example, the control unit may be configured to change the operation mode of the first sensor module/unit in case of an abnormality is detected by the abnormality detector only for a predetermined time interval (and to return to the previous operation mode after the predetermined time interval). Preferably, the predetermined time interval may be set in accordance with the time it takes the capsule endoscope to pass through the organ segment where the abnormality was found. Thus, the change of the operation mode is optimized.

According to another preferred embodiment, the control unit the control unit is configured to change the operation mode of the first sensor module/unit in case a specific (anatomical) location of the capsule endoscope is determined by the location detector. In particular, the control unit may be configured to adapt the resolution or illumination brightness and/or to increase the frame rate or alter the mode of operation of the capsule endoscope in any other way in order to achieve more accurate results in a specific location of the gastrointestinal tract. For example, If the esophagus, stomach and duodenum have recently been investigated endoscopically, by e.g. esophagogastroduodenoscopy, images from the esophagus, stomach and duodenum are not of clinical interest for the capsule endoscope. Therefore, it is advantageous to maintain a mode of operation of low energy consumption while the capsule is in the esophagus, stomach or duodenum and to change the mode of operation of the first sensor module/unit or any other module/unit of the capsule endoscope when the location detector determines the (anatomical) location of the capsule endoscope to be in other gastrointestinal organ segments such as the jejunum or ileum, which have not yet been investigated by a preceding esophagogastroduodenoscopy.

In other words, the capsule endoscopy system according to the disclosure solves the problem that a part of the energy is consumed by acquiring and processing image/sensor data that are redundant to other examinations, leaving only a fraction of energy to be consumed by acquisition and processing of image/sensor data in gastrointestinal organ segments of interest. The quality of image/sensor data with from a gastrointestinal organ segment of interest is thus limited due to the limited energy left. Since the size of the capsule is very limited, the size of the battery is also limited. Thus, even though data storage components are becoming smaller and can store larger amounts of data, it is not desirable to expand the amount of data generated excessively, as not only storage consumes energy, but especially wireless transmission through tissue is very energy-consuming. Hence, it is disadvantageous to produce many data on inconspicuous/normal sites, e.g. redundant or meaningless/irrelevant data or high-resolution data, and too little data on potentially pathological sites. Thus, the selectively executed change of the operation mode improves the quality and the information value of the acquired data, especially those data from a gastrointestinal organ segment of interest.

According to a preferred embodiment, the data transmission unit may be configured to send a notification (preferably together with the sensor data acquired with the first sensor module/unit related to the abnormality and/or the relation between the sensor data acquired with the first sensor module/unit and/or the (anatomical) location to the external processing device, in particular the extracorporeal onbody device in case an abnormality is detected by the abnormality detector. Thus, a physician can be quickly made aware of the abnormality and (manually) change the control of the capsule endoscope, in particular change the operation mode of the first sensor module/unit. This has the advantage that, if necessary, the abnormality can be reacted to immediately.

According to another preferred embodiment, the data transmission unit may be configured to send a notification (preferably together with the sensor data related to the (anatomical) location of the capsule endoscope to the external processing device, in particular the extracorporeal onbody device in case an abnormality is detected by the abnormality detector. Thus, a physician can be quickly made aware of the (anatomical) location of the capsule endoscope and intervene in the procedure as required. For example, if a capsule endoscope used to inspect the small bowel reaches the colon, the investigation is complete and the patient can be released from the procedure without the risk of premature procedure interruption and related loss of data. Or, as another example, the physician or medical staff can conduct a defined step of the procedure such as the administration of a marker substance when the capsule endoscope reaches a certain gastrointestinal organ segment of interest like the jejunum in case of gut barrier evaluation using a fluorescence marker in conjunction with fluorescence sensor module/unit.

According to a preferred embodiment, the capsule endoscopy system may comprise a patient-worn onbody device as the external processing device and a remote device. The capsule endoscope is configured to communicate with the patient-worn onbody device, that is, to transmit data to the patient-worn onbody device via a (wireless) downlink and/or to receive data from the patient-worn onbody device via a (wireless) uplink, in particular by (long-wave) radio transmission. The patient-worn onbody device is configured to communicate with the remote device via a communication link, that is, to transmit data to the remote device and/or to receive data from the remote device by wire or wirelessly, e.g. by data wire, USB, WIFI, mobile data or a combination of different communication technologies/modes. The remote device may be a device capable of receiving (and/or sending) data, e.g. a smartphone, an app, a cloud or the like.

In other words, the onbody device is interposed in the data transfer and is located in the immediate vicinity, i.e. on the patient's body or close proximity to the patient. As there are significant limitations in terms of data transmission when sending data through tissue, in particular in terms of transmission speed through tissue or energy demand, it is advantageous to first transmit the data only over as short a distance as possible and from there, since there are no longer such strict restrictions/limitations as in the case of transmitting through tissue, to further transmit it in another way. Further, the physician does not have to be in direct vicinity of the patient for the entire duration of the capsule endoscopy, which is several hours, but is still able to react to the acquired sensor data and to influence the operation of the capsule endoscope at any time. Especially, in case an abnormality is detected by the abnormality detector, the physician is able to react immediately by using the remote device.

The aspect of the capsule endoscopy system comprising a patient-worn onbody device and a remote device may also be independent from the aspect of the capsule endoscope comprising a location detector and/or an abnormality detector and/or an association unit or the first sensor module/unit being an imaging system.

According to a preferred embodiment, the capsule endoscopy system may comprise an extracorporeal sensor unit as an extracorporeal reference device, and a processing device. The capsule endoscope comprises an intracorporeal sensor unit for acquiring sensor data inside a human body. This intracorporeal sensor unit may be the first sensor module/unit, the abnormality detector, the location detector or any other sensor unit inside or as part of the capsule endoscope. The (at least one) extracorporeal sensor unit serves as reference device for acquiring sensor data outside the human body, in particular in spatial proximity to the human body / capsule endoscope. The processing device is configured to determine a sensor parameter by comparing/analyzing the acquired sensor data of the intracorporeal sensor unit and the at least one extracorporeal sensor unit. Thus, a sensor value/parameter acquired inside the human body / capsule endoscope can be corrected by the comparative measurement of the at least one extracorporeal sensor unit, so that any parameter changes outside the body that would constitute an artifact to the sensor parameter inside the human body / capsule endoscope can be eliminated and do not falsify the result. Since the capsule endoscope is worn for several hours and during the wearing also a movement of the body is advantageous, a change of the environment, e.g. resulting from the movement of the body, can thus be taken into account, so that changing environment conditions do not distort the measured sensor data and more accurate results are obtained. In particular, as it is desirable to monitor the function of the gastrointestinal tract especially during movement of the patient, for example a changing ambient temperature, a changing body temperature or a changing ambient pressure due to varying altitude or the like do not falsify the correctness or informative value of the measurements acquired by the capsule endoscopy system according to the disclosure. Thus, it is avoided to initiate unnecessary or even harmful clinical actions based on incorrect/incomplete data.

In other words, the capsule endoscopy system does not only record certain parameters intracorporeally but also extracorporeally, in order to be able to take into account any resulting falsifications in the intracorporeal measurement result due to environmental factors.

The aspect of the capsule endoscopy system comprising an extracorporeal sensor unit as reference device and a processing unit may also be independent from the aspect of the capsule endoscope comprising a location detector and/or an abnormality detector and/or an association unit or the first sensor module/unit being an imaging system.

According to the preferred embodiment, the intracorporeal sensor unit and the extracorporeal sensor unit are pressure sensors, preferably with an internal pressure reference (absolute pressure sensors). That is, the extracorporeal sensor unit is a pressure sensor and configured to measure the atmospheric pressure at the location of the patient. Hence, it is possible to calculate the intracorporeal pressure acquired by the intracorporeal sensor unit that is solely attributable to the body functions by subtracting the atmospheric pressure from the intracorporeal pressure reading.

According to a preferred embodiment, the intracorporeal sensor unit and the extracorporeal sensor unit are temperature sensors. That is, the extracorporeal sensor unit is configured to measure the temperature at the location of the patient or the body temperature of the patient. Hence, the simultaneous measurement of the intracorporeal temperature and the extracorporeal temperature it is possible to determine the temperature gradient between inside and outside of the human body. In other words, the intracorporeal temperature can be put into context by considering the extracorporeal temperature. For instance, if the patient is exposed to a high environmental temperature, a high reading of the intracorporeal temperature can be clinically attributed to environmental factors rather than a pathology.

According to a preferred embodiment, the intracorporeal sensor unit and the extracorporeal sensor unit are orientation sensors, in particular accelerometer, preferable 3-axis accelerometer, and magnetometer, preferably 3-axis magnetometer. That is the extracorporeal sensor unit is configured to measure an orientation of the patient with respect to the gravity field vector and/or an external magnetic field such as the magnetic field of the earth. Hence, the orientation of a onbody device as a reference device being fixed to the (trunk of the) patient relative to the gravity vector by measuring acceleration and/or a magnetic field e.g. of the earth by measuring the magnetic field vector, may serve as reference point for an intracorporeal sensor for acceleration and magnetic field vector. By subtracting both measurements, the orientation of the capsule endoscope relative to the human body can be deducted, irrespective of posture, movement and orientation of the human body with respect to its environment.

According to a preferred embodiment, the capsule endoscopy system may be configured such that the intracorporeal sensor unit and the extracorporeal sensor unit conduct the measurement of the parameters simultaneously or time-delayed within a predetermined time interval. Preferably, the predetermined time interval may be chosen in dependence of the measured sensor parameter. Since certain parameters change only very slowly, others rather quickly, it makes sense to select the sampling interval depending on the parameter. In particular, the predetermined time interval may be chosen such that the measurements are in such a proximity in time to each other, that a change in sensor parameter is not significant. Thus, accurate measurements can be guaranteed. For example, the predetermined time interval of a first parameter, e.g. temperature and or pressure, may be greater than the predetermined time interval of a second parameter, e.g. orientation.

### Brief Description of the Drawings

The present disclosure is explained in more detail below on the basis of preferred embodiments using figures. The figures are of a schematic nature and intended to improve the understanding of the disclosure. Same elements are referenced to with the same reference signs.
Fig. 1 illustrates a first embodiment of a capsule endoscopy system comprising a endoscope capsule according to the present disclosure.
Figs. 2 to 4 illustrate exemplary embodiment of the endoscope capsule.
Fig. 5 illustrates a second embodiment of the capsule endoscopy system.
Fig. 6 illustrates a third embodiment of the capsule endoscopy system.

The disclosure is directed to a capsule endoscopy system comprising a capsule endoscope 1. The capsule endoscope 1 is preferably configured to be swallowable by a patient for passing through a gastrointestinal tract of a human body of the patient. In particular, the capsule endoscope 1 is shaped like a pill.

The capsule endoscope 1 comprises a first sensor module/unit 2, in particular an imaging system, preferably a camera device and an illumination source, for acquiring sensor data, in particular images of an inner surface, of the gastrointestinal tract of the patient.

The capsule endoscope 1 comprises a location detector 3 being configured to track a location, preferably an anatomical location, of the capsule endoscope 1. That is, the anatomical location (in contrast to a Cartesian location) refers to the location along the anatomy of the gastrointestinal tract, such as the organ segment (mouth, esophagus, stomach, duodenum, jejunum, ileum, colon, rectum) or the position within such organ segments, i.e. the travel distance within the organ.

The capsule endoscope 1 comprises an association unit 4. The association unit 4 is configured to associate the sensor data acquired with the first sensor module/unit 2, in particular the acquired images, with the (anatomical) location of the capsule endoscope 1. The association unit 4 may be a data processing unit that establishes the relation between the (anatomical) location determined by the location detector with sensor data acquired with the first sensor module/unit 2 by e.g. attributing identifiers, time stamps or grouping data accordingly.

In other words, the capsule endoscope 1 according to the disclosure does not only measure sensor data with its first sensor module/unit 2 which have to be analyzed at a later stage, but already establishes the relation between the sensor data acquired with the first sensor module/unit 2 and the (anatomical) location determined with the location detector 3.

Preferably, the location detector 3 may be a near-field imaging system 31, a movement detector/tracer 32, an accelerometer 33, a magnetometer 34, a pH sensor 35, a bile detector/sensor 36 or a temperature sensor 37. Alternatively or additionally, the location detector 3 may be a data processing unit 38 being configured to determine the (anatomical) location from the sensor data acquired with the first sensor module/unit 2, in particular form the images of the inner surface of the gastrointestinal tract. The location detector 3 may also be a combination of said sensor types and/or said data processing unit.

According to a further aspect, which may be present in combination with or independently of the above-described aspect of the capsule endoscope comprising a location detector 3, the capsule endoscope 1 may comprise an abnormality detector 5.

The abnormality detector 5 is configured to detect an abnormality in the gastrointestinal tract. The abnormality may be indicative of a pathology.

Preferably, the association unit 4 may be configured to associate the sensor data acquired with the abnormality detector 5 with the sensor data acquired with the first sensor module/unit 2, in particular the acquired images.

Preferably, the association unit 4 may be configured to additionally associate the sensor data acquired with the abnormality detector 5 with the anatomical location of the capsule endoscope 1 determined with the location detector 3.

That is, the association unit 4 establishes a relation between the sensor data acquired with the first sensor module/unit 2, in particular images, and the sensor data concerning the abnormality, and preferably additionally with the (anatomical) location of the abnormality.

Preferably, the association unit 4 may be configured to only associate the relation between the (anatomical) location of the capsule endoscope 1 determined by the location detector 3 and the sensor data acquired with the first sensor module/unit 2 in case of a detection of an abnormality.

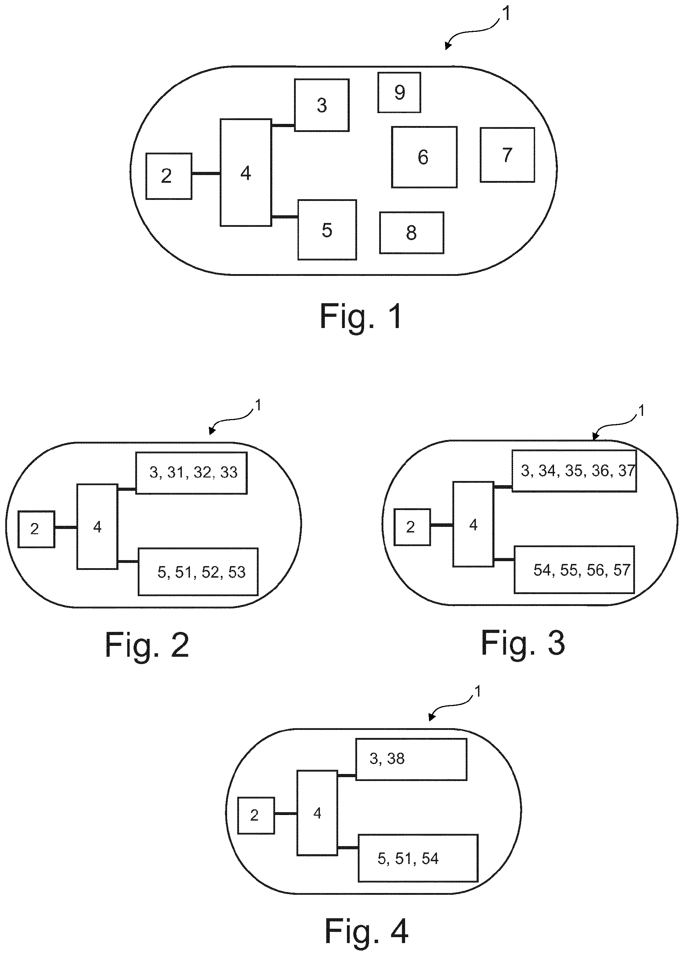

According to an exemplary embodiment of the capsule endoscope 1 (see Fig. 2), the capsule endoscope 1 at least comprises an imaging system as the first sensor module/unit 2, a temperature sensor 31 as the location detector 3 and a blood detector/sensor 51 as the abnormality detector 5.

Alternatively or additionally, the capsule endoscope 1 may also be equipped with a bile detector/sensor 32 and/or pH detector/sensor 33 as the location detector 3. With the bile detector/sensor 32 and/or pH detector/sensor 33 it is possible to determine the passage of the pylorus by sensing the exposure to bile juice either through a bile detector (e.g. optical sensor sensitive to colorants in the bile) or the increase of pH value relative to the intragastic pH value. In combination with the temperature sensor 31, an excretion of the capsule endoscope 1 is indicated through a drop in temperature after leaving the patient's body.

Alternatively or additionally, the capsule endoscope 1 may also be equipped with a gas sensor 52 and/or the fluorescence sensor 53 as the abnormality detector 5. With thegas sensor 52 and/or the fluorescence sensor 53 it is possible to determine abnormal concentrations of markers and/or other substances in the lumen of the gastrointestinal tract that can be considered abnormal, e.g. certain gas levels of methane or hydrogen that could be indicative of small intestinal bacterial overgrowth or other pathologies.

According to another exemplary embodiment of the capsule endoscope 1 (see Fig. 3), the capsule endoscope 1 at least comprises an imaging system as the first sensor module/unit 2, a movement detector/tracer 34, preferably in form an accelerometer 35, preferably a 3-axis accelerometer, and a magnetometer 36, preferably a 3-axis magnetometer, as the location detector 3 and a data processing unit 54 as the abnormality detector 5. The imaging system may be configured as near-field imaging system.

Alternatively or additionally, the capsule endoscope 1 may also be equipped with a data processing unit 37 as the location detector 3 by analyzing subsequent frames of the first sensor module/unit 2 in order to determine the travel distance in particular in the small bowel.

Alternatively or additionally, the capsule endoscope 1 may also be equipped with a movement detector/tracer 55, preferably in form an accelerometer 56, preferably a 3-axis accelerometer, and a magnetometer 57, preferably a 3-axis magnetometer, as the abnormality detector 5 by obtaining movement patterns with respect to the gravity vector and an extracorporeal magnetic field such as the earth magnetic field in order to identify abnormalities in gastrointestinal motility.

According to another exemplary embodiment of the capsule endoscope 1 (see Fig. 4), the capsule endoscope 1 at least comprises an imaging system as the first sensor module/unit 2, the data processing unit 37 as the location detector 3 and the blood sensor 51 as the abnormality detector 5.

Alternatively or additionally, the capsule endoscope 1 may also be equipped with the data processing unit 54 as the abnormality detector 5 for analyzing the content of the images acquired by the first sensor module/unit 2 for the detection of abnormalities.

Further, the capsule endoscope 1 may also be equipped with a near-field imaging system, the temperature sensor 31, the bile sensor 32, the pH sensor 33, the movement detector/tracer 34, preferably in form of the combination of an accelerometer 35, preferably a 3-axis accelerometer, and the magnetometer 36, preferably a 3-axis magnetometer, or the data processing unit 37 as the location detector 3. The location detector 3 may also be a combination of said sensor types and/or said data processing unit.

Further, the capsule endoscope 1 may also be equipped with a bile sensor, a temperature detector/sensor, a pH detector/sensor, a near-field imaging system, the blood detector/sensor 51, a gas sensor 52, the fluorescence sensor 53, the data processing unit 54 the movement detector/tracer 55, preferably in form the accelerometer 56, preferably a 3-axis accelerometer, and the magnetometer 57, preferably a 3-axis magnetometer. The abnormality detector 5 may also be a combination of said sensor types and/or said data processing unit.

Preferably, the capsule endoscopy system may comprise a control unit 6 being configured to control an operation mode of the first sensor module/unit 2, in particular the imaging system. The operation mode may be a measurement interval of the first sensor module/unit 2, in particular a frame rate of the imaging system, or a setting of the first sensor module/unit 2, in particular a resolution or an illumination brightness of the imaging system.

Preferably, the control unit 6 is configured to change the operation mode of the first sensor module/unit 2 in case an abnormality is detected by the abnormality detector 5. In particular, the control unit 6 may be configured to adapt the resolution or illumination brightness and/or to increase the frame rate or alter the mode of operation of the capsule endoscope 1 in any other way.

Preferably, the control unit the control unit 6 is configured to change the operation mode of the first sensor module/unit 2 in case a specific (anatomical) location of the capsule endoscope 1 is determined by the location detector 3. In particular, the control unit 6 may be configured to adapt the resolution or illumination brightness and/or to increase the frame rate or alter the mode of operation of the capsule endoscope 1 in any other way.

For example, the control unit 6 may be configured to change the operation mode of the first sensor module/unit 2 in case of an abnormality is detected by the abnormality detector 5 only for a predetermined time interval (and to return to the previous operation mode after the predetermined time interval). Preferably, the predetermined time interval may be set in accordance with the time it takes the capsule endoscope 1 to pass through the organ segment where the abnormality was found.

The control unit 6 may also be configured to control an operation mode of modules of the capsule endoscope 1 other than the first sensor module/unit 2. For example, the control unit 6 may be configured to control an operation mode of the location detector 3, the association unit 4 and/or the abnormality detector 5.

Further, the capsule endoscope 1 may comprise a data transmission unit 7 for (wirelessly) transmitting, preferably in real time, the acquired data to an external processing device, in particular to an extracorporeal onbody device 10.

Further, the capsule endoscope 1 may comprise an internal data storage unit 8 for storing the acquired data. Thus, the acquired data can be stored in the internal data storage unit, that is, in capsule endoscope 1 itself, and, after the capsule endoscope 1 has been excreted, the acquired data can be read out and evaluated.

Further, the capsule endoscope 1 may comprise an energy source 9, in particular a battery, for providing the sensor modules, detector modules and/or the data storage unit 8 and/or data transmission unit 7 with energy. The energy source 9 may comprises at least one regulator device for electrical energy regulation such as for stabilization and/or device activation.

Further, the control unit 6 may also be configured to control an operation mode of the energy source 9, the internal data storage unit 8, (the transmission function of) the data transmission unit 7, and/or any other module/unit incorporated into the capsule endoscope 1.

Preferably, the data transmission unit 7 may be configured to send a notification (preferably together with the sensor data acquired with the first sensor module/unit 2 related to the abnormality and/or the relation between the sensor data acquired with the first sensor module/unit 2 and/or the (anatomical) location to the external processing device, in particular the extracorporeal onbody device 10 in case an abnormality is detected by the abnormality detector 5.

Preferably, the data transmission unit 7 may be configured to send a notification (preferably together with the sensor data related to the (anatomical) location of the capsule endoscope 1 to the external processing device, in particular the extracorporeal onbody device 10 in case an abnormality is detected by the abnormality detector 5.

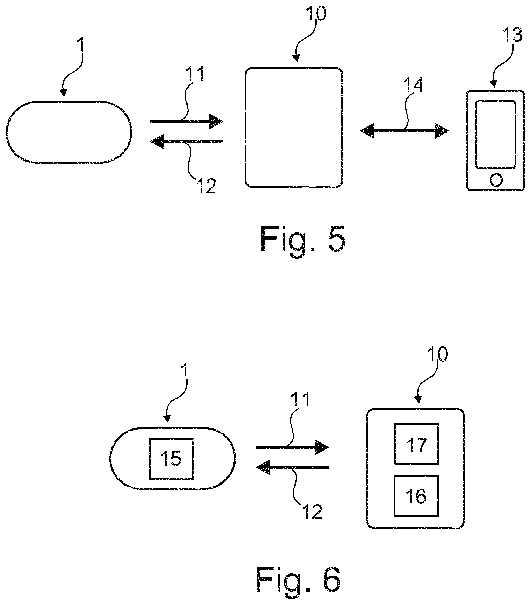

Preferably, the capsule endoscopy system may comprise a patient-worn onbody device 10 as the external processing device and a remote device 13 (see Fig. 5). The capsule endoscope 1 is configured to communicate with the patient-worn onbody device 10, that is, to transmit data to the patient-worn onbody device via a (wireless) downlink 11 and/or to receive data from the patient-worn onbody device via a (wireless) uplink 12, in particular by (long-wave) radio transmission. The patient-worn onbody device 10 is configured to communicate with the remote device 13 via a communication link 14, that is, to transmit data to the remote device and/or to receive data from the remote device by wire or wirelessly, e.g. by data wire, USB, WIFI, mobile data or a combination of different communication technologies/modes. The remote device 13 may be a device capable of receiving (and/or sending) data, e.g. a smartphone, an app, a cloud or the like.

The aspect of the capsule endoscopy system comprising a patient-worn onbody device 10 and a remote device 13 may also be independent from the aspect of the capsule endoscope 1 comprising a location detector 3 and/or an abnormality detector 5 and/or an association unit 4 or the first sensor module/unit 2 being an imaging system.

Preferably, the capsule endoscopy system may comprise an extracorporeal sensor unit 16 as an extracorporeal reference device and a processing device 17 (see Fig. 6). The capsule endoscope 1 comprises an intracorporeal sensor unit 15 for acquiring sensor data inside a human body. The intracorporeal sensor unit 15 may be the first sensor module/unit 2, the abnormality detector 5, the location detector 3 or any other sensor unit inside or as part of the capsule endoscope 1. The (at least one) extracorporeal sensor unit 16 serves as reference device for acquiring sensor data outside the human body, in particular in spatial proximity to the human body / capsule endoscope 1. The processing device 17 is configured to determine a sensor parameter by comparing/analyzing the acquired sensor data of the intracorporeal sensor unit 15 and the at least one extracorporeal sensor unit 16.

The aspect of the capsule endoscopy system comprising an extracorporeal sensor unit 16 as reference device and a processing unit may also be independent from the aspect of the capsule endoscope 1 comprising a location detector 3 and/or an abnormality detector 5 and/or an association unit 4 or the first sensor module/unit 2 being an imaging system.

Preferably, the intracorporeal sensor unit 15 and the extracorporeal sensor unit 16 are pressure sensors, preferably with an internal pressure reference (absolute pressure sensors). That is, the extracorporeal sensor unit 16 is a pressure sensor and configured to measure the atmospheric pressure at the location of the patient.

Preferably, the intracorporeal sensor unit 15 and the extracorporeal sensor unit 16 are temperature sensors. That is, the extracorporeal sensor unit 16 is configured to measure the temperature at the location of the patient or the body temperature of the patient.

Preferably, the intracorporeal sensor unit 15 and the extracorporeal sensor unit 16 are orientation sensors, in particular accelerometer, preferable 3-axis accelerometer, and magnetometer, preferably 3-axis magnetometer. That is the extracorporeal sensor unit 16 is configured to measure an orientation of the patient with respect to the gravity field vector and/or an external magnetic field such as the magnetic field of the earth.

Preferably, the capsule endoscopy system may be configured such that the intracorporeal sensor unit 15 and the extracorporeal sensor unit 16 conduct the measurement of the parameters simultaneously or time-delayed within a predetermined time interval.

Preferably, the predetermined time interval may be chosen in dependence of the measured sensor parameter.

### Reference Signs

- 1: Capsule endoscope
- 2: First sensor module/unit
- 3: Location detector
- 4: Association unit
- 5: Abnormality detector
- 6: Control unit
- 7: Data transmission unit
- 8: Data storage unit
- 9: Energy source
- 10: Onbody device
- 11: Downlink
- 12: Uplink
- 13: Remote device
- 14: Communication link
- 15: Intracorporeal sensor unit
- 16: Extracorporeal sensor unit
- 17: Processing device
- 31: Temperature sensor
- 32: Bile sensor
- 33: pH sensor
- 34: Movement detector/tracer
- 35: Accelerometer
- 36: Magnetometer
- 37: Data processing unit
- 51: Blood detector/sensor
- 52: Gas sensor
- 53: Fluorescence sensor
- 54: Data processing unit
- 55: Movement detector/tracer
- 56: Accelerometer
- 57: Magnetometer

## Claims

1. A capsule endoscopy system for examination of a gastrointestinal tract of a human body of a patient comprising a capsule endoscope (1), the capsule endoscope (1) preferably being configured to be swallowable by the patient for passing through the gastrointestinal tract, in particular the capsule endoscope (1) shaped like a pill,
the capsule endoscope (1) comprising
a first sensor module/unit (2), in particular an imaging system, preferably a camera device and an illumination source, for acquiring sensor data, in particular images of an inner surface, of the gastrointestinal tract of the patient,
a location detector (3) being configured to track a location, preferably an anatomical location, of the capsule endoscope, and
an association unit (4) being configured to associate the acquired sensor data, in particular the acquired images, with the location of the capsule endoscope.

2. The capsule endoscopy system according to claim 1, **characterized in that** the location detector (3) is
a near-field imaging system, a temperature sensor (31), a bile sensor (32), a pH sensor (33),a movement detector/tracer (34), an accelerometer (35), a magnetometer (36), or a data processing unit (37) being configured to determine the location from the acquired sensor data, in particular form the images of the inner surface of the gastrointestinal tract, or
a combination two or more of a near-field imaging system, a temperature sensor (31), a bile sensor (32), a pH sensor (33),a movement detector/tracer (34), an accelerometer (35), a magnetometer (36), or a data processing unit (37).

3. The capsule endoscopy system according to claim 1 or 2, **characterized in that** the capsule endoscope (1) further comprises an abnormality detector (5) being configured to detect an abnormality in the gastrointestinal tract.

4. The capsule endoscopy system according to claim 3, **characterized in that** the abnormality detector (5) is
a blood detector/sensor (51), a gas sensor (52), a fluorescence sensor (53), a bile detector/sensor, a temperature detector/sensor, a pH detector/sensor, a movement detector/tracer (55), an accelerometer (56), a magnetometer (57), a near-field imaging system, another detector/sensor, or a data processing unit (54) being configured to detect an abnormality based on the acquired sensor data of the first sensor module/unit (2), in particular of the acquired images of the inner surface of the gastrointestinal tract, or
a combination two or more of a blood detector/sensor (51), a gas sensor (52), a fluorescence sensor (53), a bile detector/sensor, a temperature detector/sensor, a pH detector/sensor, a movement detector/tracer (55), an accelerometer (56), a magnetometer (57), a near-field imaging system, or a data processing unit (54).

5. The capsule endoscopy system according to claim 3 or 4, **characterized in that** the association unit (4) is configured to associate the abnormality with the acquired sensor data, in particular the acquired images, and preferably additionally with the location of the capsule endoscope (1).

6. The capsule endoscopy system according to any of claims 3 to 5, **characterized in that** the association unit (4) is configured to only associate the relation between the location and the acquired sensor data in case of a detection of an abnormality.

7. The capsule endoscopy system according to any of claims 3 to 6, **characterized in that** the capsule endoscopy system further comprises a control unit (6) being configured to control an operation mode, in particular a measurement interval or a setting, of the first sensor module/unit (2), in particular the imaging system, in particular the frame rate, resolution and/or illumination brightness of the imaging system, the control unit (6) being configured to change the operation mode of the first sensor module/unit (2) in case an abnormality is detected by the abnormality detector (5), and/or the control unit (6) being configured to control an operation mode of the location detector (3), the association unit (4), and/or the abnormality detector (5).

8. The capsule endoscopy system according to any of claims 3 to 7, **characterized in that** the capsule endoscope (1) further comprises a data transmission unit (7) for transmitting the acquired data to an external processing device, in particular to an patient-worn onbody device (10), the data transmission unit (7) being configured to send a notification, preferably together with the acquired sensor data related to the abnormality and/or the relation between the acquired sensor data and/or the location, to the external processing device in case an abnormality is detected by the abnormality detector (5).

9. The capsule endoscopy system according to any of claims 1 to 8, **characterized in that** the capsule endoscopy system further comprises
a patient-worn onbody device (10), the patient-worn onbody device (10) being configured to communicate with the capsule endoscope (1) wirelessly, in particular by long-wave radio transmission, and
a remote device (13), the remote device being configured to communicate with the patient-worn onbody device by wire or wirelessly, preferably by data wire, USB, WIFI, or mobile data.

10. The capsule endoscopy system according to any of claims 1 to 9, **characterized in that** the capsule endoscopy system further comprises
an extracorporeal reference device (10), the reference device comprising a second sensor unit (16) for acquiring sensor data outside the human body, in particular in spatial proximity to the human body/first sensor unit, and
a processing device (17), the processing device being configured to determine a sensor parameter by comparing/analyzing the acquired sensor data of the first sensor module/unit (2, 15) and the second sensor unit (16).

11. The capsule endoscopy system according to claim 10, **characterized in that** the second sensor unit (16) is configured to measure an atmospheric pressure at a location of the patient and/or the second sensor unit (16) is configured to measure a temperature at a location of the patient or a body temperature of the patient and/or the second sensor unit (16) is configured to measure an orientation of the patient.

12. The capsule endoscopy system according to claim 10 or 11, **characterized in that** the capsule endoscopy system is configured such that the first sensor unit (15) and the second sensor unit (16) conduct the measurement of the sensor parameter simultaneously or time-delayed and within a predetermined time interval, the predetermined time interval being chosen in dependence of the measured sensor parameter, preferably the predetermined time interval being chosen such that the measurements are in such a proximity in time to each other, that a change in sensor parameter is not significant.
